# EUROPEAN PATENT SPECIFICATION

(11) **EP 0 610 056 B1**
(45) Date of publication and mention of the grant of the patent: **05.06.2002**
(21) Application number: 94300720.3
(22) Date of filing: 01.02.1994
(51) Int. Cl.: A61L 15/60, A61L 15/58, A61F 13/02

(54) **Hydrogel bandages**
Hydrogelverbände
Pansements à base d'hydrogel

(30) Priority: 02.02.1993 US 12215; 04.10.1993 US 131040
(43) Date of publication of application: 10.08.1994
(73) Proprietor: Johnson & Johnson Consumer Companies, Inc., Skillman, New Jersey 08558 (US)
(72) Inventor: Partyka, Doris, Milltown, NJ 08850 (US); Taylor, Thomas R., Mercerville, NJ 08619 (US); Kundel, Nikhil K., Pascataway, NJ 08854 (US)
(74) Representative: Fisher, Adrian John

(56) References cited:
- EP-A- 0 107 376
- EP-A- 0 190 814
- EP-A- 0 304 536
- EP-A- 0 424 165
- EP-A- 0 450 671
- EP-A- 0 455 324
- EP-A- 0 536 875
- GB-A- 987 779

## Description

This invention relates to hydrogel bandages.

The use of hydrogels in the treatment and management of burns and wounds is well known in the art. Hydrogel dressings are further desirable because wound exudate does not generally dry and consolidate with hydrogels or hydrogel laminates. Consequently, removal of a hydrogel dressing is usually neither painful nor detrimental to the healing process. It has been suggested that hydrogel dressings may be particularly desirable for treatment of burns because they may accelerate healing. Although the mechanism by which hydrogels stimulate healing is not fully elucidated, it is documented that the high water content of hydrogels enables them to effect an immediate cooling of the wound surface and to sustain the reduced temperature for up to six hours. Davis, et al., "A New Hydrogel Dressing Accelerates Second-Degree Burn Wound Healing," Poster Presentation, *Wound Healing Society First Annual Meeting,* Galveston, Tx, Feb. 6, 1991. In addition, water swollen hydrogels may provide a cushioning effect that helps protect the burn or wound from physical trauma.

U.S. Patent 4,438,258 relates to hydrogels which may be used as interfaces between damaged skin tissue and its external environment. As disclosed therein, hydrogels may be polymerized about some type of support, such as a mesh of nylon, used as an unsupported film, spun in fibers and woven into a fabric, or used as a powder. Further, hydrogels may be used to provide a controlled release of medical composition.

U.S. Patent 4,552,138 discloses a wound dressing material of at least one layer of polymeric, hydrophilic gel wherein the gel is cross-linked and acetalized with formaldehyde. As disclosed therein, a gel film may be formed by spreading a pre-crosslinked gel on an auxiliary carrier, drying and at the same time cross-linking the same by heat treatment. As used in this disclosure, uncrosslinked polyvinyl alcohol is dissolved in water, acidified, preferably by hydrochloric acid, and combined with an aqueous formaldehyde solution and left to react or pre-crosslink at 50-80°C for several hours to obtain a gelatinous mass wherein no further free aldehyde can be detected. Such gel films may be placed on the wound as such, but they are preferably processed to a laminated product with one or more carrier materials and used in this form. The carrier layers are laminated into or onto the pre-crosslinked gel layer and may be further cross-linked to bond more firmly with the gel.

U.S. Patent 4,554,317 discloses a synthetic hydrophilic membrane prepared by graft polymerization of hydrophilic monomers with a polyurethane substrate. This membrane is particularly useful as a wound covering material. In one embodiment of this invention, the graft polymerization is initiated by X-ray of gamma radiation or an initiator such as a cerium salt.

EP-A-0 107 376 discloses a tacky, non-rigid transparent and absorbent dressing comprising a layer of cross-linked polyvinylpyrrolidone gel containing about 75-85% water. The dressing may be prepared by dissolving between 15% and 25% by weight of polyvinylpyrrolidone in water and cross-linking the polyvinylpyrrolidone by means of ionizing radiation.

U.S. Patent 4,567,006 discloses a moisture vapor permeable, adhesive surgical dressing comprising a continuous film of a hydrophilic polymer. Such a dressing is suitable for use on moist wounds because it allows water to evaporate rapidly from the wound area in the presence of an excess of exudate but, as the amount of exudate diminishes, so does the rate of evaporation. The resulting amount of exudate is enough to keep the wound moist without causing blistering of the dressing.

U.S. Patent 4,798,201 discloses a surgical dressing consisting essentially of a film which carries an adhesive layer for securing the dressing to the body. This dressing is also suitable for use on exuding wounds.

U.S. Patent 4,407,846 discloses a method of producing a hydrophilic membrane from a polyethylene base film by first irradiating the film of thickness not more than 150 µm with ionizing radiation in air or an oxygen atmosphere. Then, without additional radiation, acrylic acid and/or methacrylic acid present in the form of an aqueous solution is grafted onto the irradiated film.

U.S. Patent 3,669,103 discloses a flexible support adapted to be caused to conform to a surface of a body, wherein the support confines a dry, solid, water-swellable, water-insoluble polymeric sorbent to absorb aqueous fluid elaborated by the body to which the support is applied. The polymer sorbent is a lightly cross-linked polymer.

U.S. Patent 4,192,727 discloses a polyelectrolyte hydrogel and method for preparing the same. The polyelectrolyte hydrogel is formed by exposing an acrylate salt and acrylamide to a controlled intensity and dose of ionizing radiation to effect simultaneous cross-linking and polymerization thereof. The resulting hydrogel is an insoluble hydrophilic copolymer which can contain or absorb aqueous fluid.

U.S. Patent 4,646,730 discloses a color stabilized sulfadiazine hydrogel dressing comprising a non-rigid layer of cross-linked polyvinylpyrrolidone gel having incorporated therein at least 0.1% by weight of silver sulfadiazine, which gel has been exposed to electron beam radiation and which gel also contains a color stabilizing amount of magnesium trisilicate.

U.S. Patent 4,750,482 discloses a wound or burn dressing comprising a web-like substrate coated with a layer of crosslinked, water-insoluble, hydrophilic elastomeric, pressure-sensitive adhesive gel of a gel-forming, water-soluble polymer derived from repeating units, predominantly of vinylpyrrolidone, polyethylene glycol wherein the cross-linked gel is formed by radiation cross-linking of a solution or dispersion of the polymer in the plasticizer and water. The gel retains the plasticizer within a cross-linked three-dimensional matrix of the polymer.

EP-A-0 304 536 discloses an occlusive wound dressing comprising an adhesive layer, a fabric layer bonded to the adhesive layer, a hydrophilic absorbent polymeric layer applied to the fabric layer, and at least one occlusive backing layer. The hydrophilic absorbent polymeric layer of this dressing is applied by pouring a monomer solution onto the fabric layer and thereafter curing to yield the polymeric layer.

Japanese Patent Application No. 57-7414 issued in the name of Saburo Otsuka and assigned to Nitto Electric Ind. KK, discloses a medicinal plaster formed by spraying or spreading a solution or dispersion containing a monomer, a medicine, a releasing aid for medicine, etc., on the surface of a tacky layer formed on a support, and then irradiating it with UV or ionizing radiation.

U.S. Patent 4,871,490 discloses a method of manufacturing hydrogel dressing from synthetic and natural polymers by radiation cross-linking. The method involves an aqueous solution comprising 2-10% by weight polyvinylpyrrolidone, no more than 3% by weight or agar and 1-3% by weight of polyethylene glycol. The solution is poured into a mould to shape the dressing. The mould is then tightly closed and subjected to an ionizing radiation does in the range of 25-40 KGy.

At present, there are a few commercially available hydrogel dressings, for example, SECOND SKIN® from Speneo, VIGILON® from C.R. Bard and CLEARSITE® from New Dimensions in Medicine. Unfortunately, these have been structured in an unconventional way that fails to provide the convenience and familiarity that end users have grown accustomed to in using wound dressing such as adhesively attached finger bandages and patches.

One major reason for the difficulty in presenting a hydrogel dressing in a conventional format is the difficulty in handling the structurally weak gel and the incorporation of such a dressing into a high speed bandage producing process. Still another difficulty is that the gel is inherently incompatible with pressure sensitive adhesives i.e., the high water content of the gel precludes a gel dressing from securely adhering to a pressure sensitive surface. These problems have been addressed to a degree in US-A-5 480 717 and US-A-5 674 346 (see also EP-A-0 604 103). Described therein is a gel laminate capable of adhering to a pressure sensitive adhesive. The laminate comprising a substrate carrying a layer of polymeric adhesive. The gel is coated onto the polymeric adhesive and then the polymer of the gel is co-polymerized with the polymer of the adhesive; i.e, the gel and adhesive are united at the interface by covalent bonding. The gel laminate may then be affixed to a pressure sensitive adhesive coated bandage backing by adhering the substrate to the pressure sensitive adhesive.

While, in the main, the above-described system works well, to some degree the need for multiple layers makes production more complex and the need to select hydrogels and adhesives capable of co-polymerizing creates some constraints on the choice of materials. Accordingly, it is desirable to provide alternative methods and products for affixing a hydrogel dressing to an adhesive coated bandage backing.

### Summary of the Invention

In accordance with this invention, a hydrogel dressing is provided which is suitable for adhering to an adhesive coated backing of a bandage and which is simple in construction and will allow for the use of essentially any crosslinkable hydrogel polymer.

Specifically, the dressing comprises a layer of hydrogel comprising a crosslinkable polymer and water. The hydrogel layer is in face-to-face relationship with a substrate, which substrate has a hydrogel facing surface and an opposed surface. The hydrogel facing surface comprises anchoring projections extending from the substrate and embedded into the hydrogel. The opposed surface is hydrogel occlusive and prevents any substantial amount of hydrogel from passing therethrough. In accordance with the teaching of this invention, the polymer of the hydrogel is crosslinked, in situ, on the hydrogel facing and thereby anchors the hydrogel to the surface by means of the anchoring projections.

In a specific embodiment of this invention the substrate comprises a laminate of a polymeric film as the hydrogel occlusive surface and a fibrous fabric on the hydrogel facing surface. Projecting fibers from the fibrous fabric provide the anchoring projections. Since most fabric comprises a network of fibers or bundles of fiber e.g., yarns, they inherently exhibit an irregular surface pattern of raised portions and intersections. The raised portion may also serve- in combination with projecting fibers or alone, as the anchoring projections. In fact, any feature extending from the surface of the substrate toward the gel may suffice to anchor the gel to the substrate after in situ crosslinking.

In a further embodiment of this invention the dressing is positioned upon a pressure sensitive adhesive coated bandage backing. The dressing is adhered to the backing by adhesion between the hydrogel occlusive surface of the dressing and the pressure sensitive coating.

### Brief Description of the Drawings

Figure 1 is an enlarged, schematic cross-sectional view of a portion of a hydrogel dressing embodying the teachings of this invention;
Figure 2 illustrates, in perspective view an adhesively attachable bandage having positioned thereon the hydrogel dressing of Figure 1; and
Figure 3 is a cross-sectional view of the bandage of Figure 2, taken through line 1-1 thereof.

### Detailed Description of the Invention

This invention relates to hydrogel dressings, useful in absorbent products such as bandages. Hydrogels are three-dimensional networks of hydrophilic polymers, generally covalently or ionically cross-linked, which interact with aqueous solutions by swelling to some equilibrium value. These cross-linked gels are generally formed from synthetic polymers such as polyvinylpyrrolidone; polyethyleneoxide; polyacrylate, polymethacrylate, and polyacrylamide polymers and copolymers, multivalent alcohols (such as polyvinylalcohol); biopolymers (such as gelatin, agar); or combinations thereof. For the purpose of preparing bandages or wound dressings, as in this invention, additional agents may be incorporated into the hydrogel, such as color stabilizers or coloring agents, and medicaments such as antibacterial agents.

A preferred hydrogel for use in this invention is crosslinked polyvinylpyrrolidone (PVP). Best results have been achieved using PVP polymers having a viscosity average molecular weight of 100,000-1,000,000 and preferably 200,000-900,000 An especially preferred PVP polymer is PVP K-90, available from GAF Corporation, Wayne, New Jersey, having a viscosity average molecular weight of 700,000. The viscosity average molecular weight was derived by the method described by W. Scholtan, Makromol Chem., 7, 209 (1951) and J. Hengstenberg et al. Makromol Chem., 7, 236 (1951). The hydrogel preferably comprises 5 to 40, preferably 10 to 30, weight % of the polymer complemented by 60 to 95, preferably 70 to 90, weight % water. If the molecular weight of the PVP is too high, solution viscosities become too high to work with. If too low, effective crosslinking is difficult to achieve.

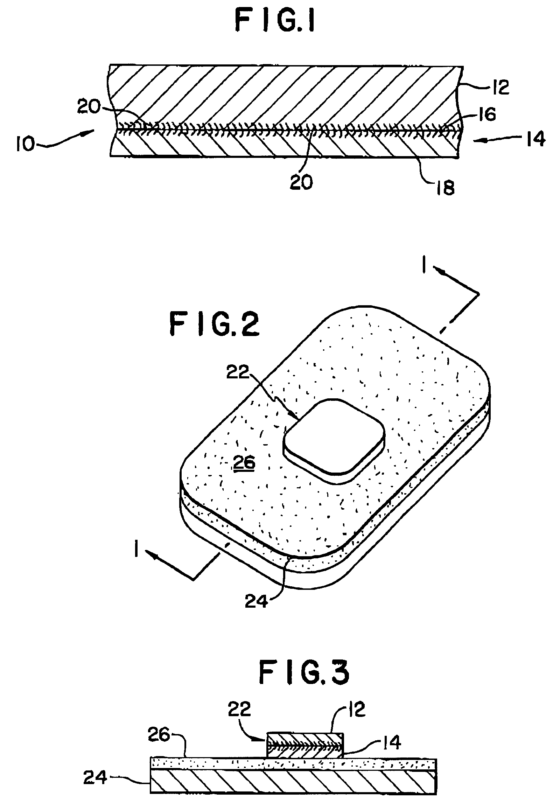

Referring now to Figure 1 illustrated there schematically is a cross-sectional view of a hydrogel dressing 10 embodying the teachings of this invention. A layer of hydrogel 12 is coated onto the surface of a substrate 14 before the crosslinkable polymer of the hydrogel is crosslinked. The substrate 14 comprises a hydrogel facing surface 16 and an opposed surface 18. The substrate 14 is chosen such that the hydrogel facing surface presents anchoring projections 20 extending into the coating hydrogel layer 12. The opposing surface 18 is chosen so as to provide a hydrogel occlusive surface which will prevent the passage of any substantial quantity of hydrogel therethrough.

We have discovered that by producing the dressing described in general terms above and then crosslinking the hydrogel polymer in situ on the surface 16, the hydrogel layer, by virtue of the cooperation between the projections 20 and the in situ crosslinked polymer, is resistant to delamination from the substrate 14 to the extent satisfactory for producing a dressing for an adhesive bandage. At the same time, the hydrogel occlusive surface may be applied to an adhesive coated backing to form a bandage. Because of the occlusive nature of the opposed surface of the substrate, the dressing will adhere to pressure sensitive adhesive and resist delamination. It should be understood that, absent such an occlusive surface, the water present in high concentrations in the hydrogel would preclude such adherence and, heretofore, has represented a great drawback in attempts to produce adhesively attached bandages employing hydrogel dressings.

The substrate meeting the requirements set out above may take many alternative forms, as can be understood by one skilled in the art from the teachings set out herein. The criteria for the material making up the occlusive surface is that it must be occlusive to the extent that hydrogel is prevented from interfacing with adhesive, it must be adherent to the pressure sensitive adhesive of a coated bandage; it must be non-toxic and safe to use in conjunction with wounds; and it is preferably flexible and body conformable to the extent required for bandages. Film-like materials such as polyolefin e.g., polyethylene or polypropylene, polyethylene vinyl acetate; or even other occlusive films such as waxes or cellophane may be employed. A material of choice is a block copolymer of polybutylene terephthalate and soft segment polyether glycols sold by the DuPont company as Hytrel. Primarily for processing reasons, the occlusive surface material should preferably be present in a thickness of from about 0.025-0.127 mm (1-5 mil) and preferably from about 0.051-0.102 mm (2.4 mil) in thickness.

It is envisioned that the hydrogel facing surface of the substrate 14 is provided with the anchoring projections 20 by incorporating, onto the occlusive surface, materials which will provide such suitable projections and generally, will be in the form of fibers. Such fibrous materials as rayon, polyester, wood pulp, cotton, cotton linter, wool or combinations thereof may all be used. While such fibers may be merely affixed to the material of the occlusive surface in a random fashion, it is preferred that the fibers are in the form of a paper, board or fabric, (woven or non-woven) and either coated with the material making up the occlusive surface or laminated thereto.

In whatever way the fibrous material is provided, it is preferred that the layer of fibers produce a thickness of about 0.127-0.381 mm (5-15 mil) and preferably from about 0.203-0.305 mm (8-12 mil). The fiber lengths may preferably vary from about 0.8-76.2 mm (1/32-3 inch) and more preferably from 12.7-50.8 mm (1/2-2 inch). The fibers denier (in denier per filament) may vary preferably from about 0.5 to about 2.5 and more preferably about 1 to about 2. Fiber layers having a weight of about 0.034-0.120 kg/m² (1-3 ounce/yard²) are preferred.

The dressing is, constructed in accordance with the teachings above, with the polymer of the hydrogel in the pre-crosslinked state. As taught herein, the hydrogel is then crosslinked, in situ, on the hydrogel facing surface of the substrate. Such crosslinking may be accomplished by means well-known in the art including for example, ionizing irradiation or chemical crosslinking.

Chemical crosslinking may be accomplished by virtue of the pendent hydroxy group present on the hydrogel polymers. Accordingly, difunctional or polyfunctional agents that condense with organic hydroxyl groups may be employed as crosslinking agents. These include various aldehydes such as formaldehyde acetalaldehyde and glutaraldehyde; maleic acid and oxalic acid; dimethyl urea; polyacrolein; diisocyanate; divinyl sulfate and ceric redox systems.

Electron beam irradiation is the preferred type of ionizing irradiation. The suitable dose will, of course, depend upon the nature of the polymer(s), and can be determined by one skilled in the art. Tests suggest that the electron beam irradiation dose should preferably be at least about 2.0 Mrads and no more than about 4.0 Mrads. In a preferred embodiment, the electron beam irradiation is applied in two doses. Dose rates of 2.0 and 2.5 Mrads, 2.5 and 2.5 Mrads, 3.0 and 2.5 Mrads and 3.5 and 2.5 Mrads have been shown to directly adhere the polymer to the substrate.

Irradiating the hydrogel dressing in two doses also provides a manufacturing benefit. After the first dose of ionizing irradiation, the hydrogel layer is partially cross-linked and has sufficient strength to be die cut into the desired shape and size. These die cut dressings may be processed into bandages using conventional techniques and then packaged in hermetically sealed containers, such as foil packs. The sealed containers are then subjected to the second dose of irradiation, which fully cross-links the hydrogel and sterilizes the product.

Referring to Figures 2 and 3, shown therein is such a die cut dressing 22 placed on a bandage backing 24 whose surface is coated with a pressure sensitive adhesive 26. The bandage backing may comprise a thin polymeric film and preferably is an embossed thermoplastic film in accordance with the teachings described, for example, in U.S. Patents 3,484,835; 4,298,647; and 4,376,147. The adhesive 26 may be any suitable medical grade pressure-sensitive adhesive as are well-known in the art. In the specific illustrated embodiment, the dressing 22 is in the form of an "island" pad but it will be clear to one skilled in the art that other bandage configurations may embody the teachings herein.

The advantages of the invention are illustrated by the following examples:

### Comparative Example

To illustrate the difficulty encountered in employing hydrogel dressings, (even when reinforced) in pressure sensitive adhesive bandages, the following experiment was conducted. A bandage backing comprising a 0.051 mm (2 mil) thick copolyester ether elastomer (Hytrel 4778 from duPont) was coated on one side with a pressure sensitive adhesive consisting of an acrylic multipolymer emulsion adhesive (Gelva 2478 from Monsanto Corporation) at a coating weight of 0.041 kg/m² (1.2 oz/yd²). The adhesive coated substrate was placed adhesive side up, in the cavity of a two plate metal mold measuring 35.56 cm (14 inches) by 35.56 cm (14 inches). The bottom plate had a square cavity therein measuring 25.4 cm (10 inches) by 25.4 cm (10 inches) and was 1.524 mm (60 mil) deep; the top plate was flat. A hydrogel solution of the following composition was prepared:

| Component | % by Weight |
|---|---|
| Polyvinylpyrrolidone (Plasdone K-90 from GAF Corp.) | 20.00 |
| Methyl Paraben | 0.26 |
| Ethyl Paraben | 0.03 |
| Propyl Paraben | 0.05 |
| Butyl Paraben | 0.01 |
| 2-Phenoxyethanol | 0.50 |
| Water | 79.15 |

60 g of the solution was spread over the adhesive coated side of the backing in the mold. A reinforcing layer comprising a fusible fiber fabric containing polyethylene and polypropylene fibers was placed over the hydrogel. A 0.178 mm (7 mil) thick release film of polyethylene was placed over the reinforcing layer.

The top plate of the mold was placed over the bottom plate and held for a few minutes to allow the reinforcing layer to become embedded into the hydrogel. The resulting structure as then removed from the mold and then irradiated using a two-pass electron beam irradiation process. The first pass delivered a dosage of 2.0 Mrads, while the second pass delivered 2.5 Mrads. After irradiation the adhesive between the hydrogel and the backing was examined. The hydrogel layer readily peeled from the backing.

### Example 1

An adhesive coated backing identical to that of the foregoing example was placed in the same mold, adhesive side up. A substrate, conforming to the teachings of this invention was placed onto the adhesive coating fiber side up. The substrate is a non-woven fabric having coextruded onto one side as the occlusive surface, a film of low density polyethylene at a lay down of 0.068 kg/m² (2 oz/yd²). The fabric had the following composition:

| Component | % by Weight |
|---|---|
| Rayon Staple Fiber 1.5*dpf, 1.59-14.29 mm (1/16-9/16 inch) length (8171; BASF) | 68.60 |
| | |
| Polyester Staple Fiber 1.5 dpf, 38.10 mm length (Fortrel 310, Fiber Industries) | 29.40 |
| | |
| Acrylic Polymer Emulsion (Rhoplex HA-8; Rohm and Haas) | 1.73 |
| | |
| Silicone Antifoam Emulsion (Antifoam Y-30; Dow Corning) | 0.01 |
| | |
| Sodium Dioctyl Sulfosuccinate Surfactant (Deceresol OT Special, Cyanamid) | 0.08 |
| | |
| Ammonium Phosphate, Dibasic (Stauffer Chemical) | 0.18 |

| | |
|---|---|
| *Denier Per Filament | |

The fabric basis weight is 0.061 kg/m² (1.8 ounce/yard²) and was produced from the blend of 70% rayon and 30% polyester. The acrylic binder (HA-8) is applied by saturating the fabric in a padder.

60g grams of a gel composition identical to that of the foregoing example was spread over the fibrous side of the substrate. A 0.178 mm (7 mil) thick polyethylene release film was placed over the gel. The top plate of the mold was placed over the bottom plate for a few minutes to allow the embedding of the gel into the projecting fibers of the fabric surface of the substrate. The resulting structure was then removed from the mold and irradiated as described in the foregoing example.

After irradiation, the adhesion between the hydrogel and the substrate as well as the adhesion between the substrate and the pressure sensitive adhesive coating was examined. Very strong adhesion was observed and none of the layers could be readily peeled apart.

### Example 2

A gel bandage was made in accordance with the procedure of Example 1 with the exception that a different substrate was employed. The substrate of this example comprised a web of individualized fibers prepared by feeding a picker lap comprising a blend of 80% by weight, bicomponent fibers and 20% by weight of rayon fibers to a standard carding engine. The bicomponent fibers were 3 denier and 38.10 mm (1.5 inch) staple and were supplied by BASF of Charlotte, N.C. The bicomponent fibers had a polyester core with a polyethylene sheath. The rayon fibers were 1.5 denier and 39.62 mm (1.56 inch) staple and were supplied by American Enka of Enka, N.C.

The formed web of individualized fibers weighing 0.033 kg/m² (0.98 ounce/yard²) was bonded by passing the web through a thru air bonder at 10.67 m/min (35 ft/min), at a drum temperature of 150°C (300°F) using an 80 x 80 belt carrier. The bonded web was rolled up. The thru air bonder was supplied by Honeycomb Systems, Inc. of Biddeford, Maine.

The bonded rolled up web was combined in a face to face relationship with a coextruded ethylene vinyl acetate/ethylene film which was supplied by Edison Plastics of Edison, N.J. and identified as EVA 456. The film was 0.038 mm (1.5 mil) in thickness. The EVA side of the coextruded film was placed in contact with the nonwoven fabric.

The film/bonded nonwoven were combined by processing them through a belt laminator at a speed of 4.11 m/min (4.5 yds/min) under a belt tension of 276 kN/m² (40 psi) and a roll pressure of 241 kN/min (35 psi) with a temperature setting of 115°C. The belt laminator was a model MTC 101-1000 as supplied by Stork of Charlotte, N.C.

When this substrate was employed in the gel dressing as per the prior example; and tested for delamination properties, it was found to resist such delamination.

## Claims

1. A hydrogel dressing suitable for use in a pressure sensitive adhesive bandage comprising:
a layer of hydrogel, said hydrogel comprising a cross-linkable polymer and water;
said hydrogel layer being in a face-to-face relationship with a substrate, said substrate having a hydrogel facing surface and an opposed surface;
said hydrogel facing surface comprising a fibrous layer having a thickness of 0.127-0.381 mm and said hydrogel facing surface having anchoring projections extending into said hydrogel layer;
said opposed surface being hydrogel occlusive and comprising an extruded polymer film; and said hydrogel being cross-linked, in situ, on said hydrogel facing surface and thereby anchored to said surface.

2. The dressing of claim 1 wherein said fibrous layer comprises fibres selected from the group consisting of rayon, polyester, wood pulp, cotton, cotton linters, wool fibres or combinations thereof.

3. The dressing of claim 2 wherein the fibres are in the form selected from the group consisting of paper, board or fabric.

4. The dressing of claim 3 wherein the fabric is a non-woven fabric.

5. The dressing of claim 1 wherein the fibres of said fibrous layer have a length of from 0.8 mm (1/32 inch) to 76.2 mm (3 inches).

6. The dressing of claim 1 wherein the fibres of said fibrous layer have a denier per filament of from 0.5 to 2.

7. The dressing of any preceding claim wherein the fibrous layer has a thickness of 0.203 mm (8 mil) to 0.305 mm (12mil).

## Patentansprüche

1. Hydrogelverband, der für den Gebrauch in einer druckempfindlichen, haftenden Bandage tauglich ist, mit einer Lage aus Hydrogel, wobei das Hydrogel ein vernetzbares Polymer und Wasser umfaßt; wobei die Hydrogellage einem Substrat gegenüberliegend angeordnet ist und das Substrat eine dem Hydrogel zugewandte Oberfläche und eine entgegengesetzte Oberfläche aufweist; wobei die dem Hydrogel zugewandte Oberfläche eine faserstoffartige Lage mit einer Dicke von 0,127 bis 0,381 mm und Verankerungsvorsprünge aufweist, die sich in die Hydrogellage ausdehnen; wobei die entgegengesetzte Oberfläche das Hydrogel einschließt und einen extrudierten Polymerfilm aufweist; und wobei das Hydrogel an Ort und Stelle auf der dem Hydrogel zugewandten Oberfläche vernetzt ist und hierdurch in dieser Oberfläche verankert.

2. Verband nach Anspruch 1, wobei die faserstoffartige Lage Fasern aus Reyon, Polyester, Zellstoff, Baumwolle, Lintbaumwolle und Wollfasem oder deren Kombinationen umfaßt.

3. Verband nach Anspruch 2, wobei die Fasern aus Papier, Pappe oder Textilstoff gebildet sind.

4. Verband nach Anspruch 3, wobei der Textilstoff ein nicht gewebter Textilstoff ist.

5. Verband nach Anspruch 1, wobei die Fasern der faserstoffartigen Lage eine Länge von 0,8 mm (1/32 inch) bis 76,2 mm (3 inches) aufweisen.

6. Verband nach Anspruch 1, wobei die Fasern der faserstoffartigen Lage ein Denier pro Faden von 0,5 bis 2 aufweisen.

7. Verband nach einem der vorangehenden Ansprüche, wobei die faserstoffartige Lage eine Dicke von 0,203 mm (8 mil) bis 0,305 mm (12 mil) aufweist.

## Revendications

1. Pansement à base d'hydrogel approprié pour une utilisation dans un bandage adhésif sensible à la pression comprenant :
une couche d'hydrogel, ledit hydrogel comprenant un polymère réticulé et de l'eau;
ladite couche d'hydrogel étant positionnée face à face avec un substrat, ledit substrat ayant une surface faisant face à l'hydrogel et une surface opposée;
ladite surface faisant face à l'hydrogel comprenant une couche fibreuse ayant une épaisseur de 0,127 à 0,381 mm et ladite surface faisant face à l'hydrogel ayant des projections enracinantes se prolongeant dans ladite couche d'hydrogel;
ladite surface opposée étant occlusive à l'hydrogel et comprenant un film polymérique extrudé; et ledit hydrogel étant réticulé, in situ, sur ladite surface faisant face à l'hydrogel et de cette façon enraciné sur ladite surface.

2. Pansement selon la revendication 1, dans lequel ladite couche fibreuse comprend des fibres sélectionnées parmi le groupe comprenant de la rayonne, du polyester, de la pâte à bois, du coton, des linters de cotons, des fibres de laine ou des combinaisons de ceux-ci.

3. Pansement selon la revendication 2, dans lequel les fibres se présentent sous la forme sélectionnée parmi le groupe comprenant du papier, du carton ou du tissu.

4. Pansement selon la revendication 3, dans lequel le tissu est un tissu non tissé.

5. Pansement selon la revendication 1, dans lequel les fibres de ladite couche fibreuse ont une longueur allant de 0,8 mm (1/32 pouce) à 76,2 mm (3 pouces).

6. Pansement selon la revendication 1, dans lequel les fibres de ladite couche fibreuse ont un denier par filament allant de 0,5 à 2.

7. Pansement selon l'une quelconque des revendications précédentes, dans lequel la couche fibreuse présente une épaisseur de 0,203 mm à 0,305 mm.
